(19) Europäisches Patentamt / European Patent Office / Office européen des brevets

(11) **EP 3 340 859 B1**

## (12) FASCICULE DE BREVET EUROPEEN

(45) Date de publication et mention de la délivrance du brevet:
**03.07.2019 Bulletin 2019/27**

(21) Numéro de dépôt: **16770059.0**

(22) Date de dépôt: **26.08.2016**

(51) Int Cl.:
*A61B 5/00* *(2006.01)*      *A61B 5/0265* *(2006.01)*
*A61B 5/0295* *(2006.01)*      *A61B 5/0408* *(2006.01)*
*A61B 5/05* *(2006.01)*      *A61B 5/0402* *(2006.01)*
*A61B 5/08* *(2006.01)*      *A61B 5/113* *(2006.01)*
*A61B 7/04* *(2006.01)*

(86) Numéro de dépôt international:
**PCT/FR2016/052130**

(87) Numéro de publication internationale:
**WO 2017/037369 (09.03.2017 Gazette 2017/10)**

(54) **DISPOSITIF ET PROCÉDÉ DE MESURE NON-INVASIVE DU DÉBIT AORTIQUE SOUS-DIAPHRAGMATIQUE CHEZ UN PETIT MAMMIFERE DE LABORATOIRE**

VORRICHTUNG UND VERFAHREN ZUR NICHTINVASIVEN MESSUNG SUBDIAPHRAGMATISCHER AORTENSTRÖME IN EINEM KLEINEN LABORSÄUGER

DEVICE AND METHOD FOR NON-INVASIVE MEASUREMENT OF SUBDIAPHRAGMATIC AORTIC FLOW IN A SMALL LABORATORY MAMMAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.08.2015 FR 1557992**

(43) Date de publication de la demande:
**04.07.2018 Bulletin 2018/27**

(73) Titulaire: **Université Grenoble Alpes**
**38400 Saint-Martin-d'Hères (FR)**

(72) Inventeurs:
• **BACONNIER, Pierre**
**38000 Grenoble (FR)**
• **BOUCHER, François**
**38000 Grenoble (FR)**
• **GUMERY, Pierre-Yves**
**38000 Grenoble (FR)**

(74) Mandataire: **Hautier IP**
**20, rue de la Liberté**
**06000 Nice (FR)**

(56) Documents cités:
**US-A- 5 178 151      US-A1- 2008 255 468**
**US-A1- 2009 326 387**

• **FONTECAVE-JALLON J ET AL: "Detecting variations of blood volume shift due to heart beat from respiratory inductive plethysmography measurements in man", PHYSIOLOGICAL MEASUREMENT, INSTITUTE OF PHYSICS PUBLISHING, BRISTOL, GB, vol. 34, no. 9, 19 août 2013 (2013-08-19), pages 1085-1101, XP020250050, ISSN: 0967-3334, DOI: 10.1088/0967-3334/34/9/1085 [extrait le 2013-08-19]**

EP 3 340 859 B1

**Description**

**DOMAINE DE L'INVENTION**

La présente invention concerne un dispositif de mesure non-invasive du débit aortique sous-diaphragmatique chez un petit mammifère de laboratoire.

**ARRIERE PLAN DE L'INVENTION**

L'exploration cardio-respiratoire chez l'homme ou l'animal est mise en oeuvre depuis de nombreuses années.

Chez le petit mammifère de laboratoire, même si elles ont évolué au cours du temps, les techniques d'exploration cardio-respiratoire sont généralement invasives.

Chauveau et Lortet ont été les premiers à décrire, en 1860, un appareil (hémodromographe) de mesure invasive des variations de vitesse d'écoulement du sang chez un mammifère vivant.

En 1947, Timm a proposé une technique de cinématoradiographie à basse vitesse permettant d'évaluer la vitesse de déplacement d'une bulle d'un produit de contraste hydrophobe injecté dans l'artère aorte de chiens et de chats. Cette technique a par la suite été améliorée avec l'utilisation de la cinématographie à haute vitesse permettant de mesurer la vitesse de déplacement de globules lipidiques dans des vaisseaux suffisamment transparents. Cette technique s'est révélée applicable sur l'aorte de lapin [1].

La première utilisation d'un débitmètre électromagnétique pour mesurer le débit sanguin aortique *in vivo* a été réalisée en 1956 [2] alors que les premières méthodes de mesure de débit par dilution de colorant ou par thermodilution commençaient à faire leur apparition dans les études sur gros mammifères ou chez l'homme. Ces dernières ne fournissent qu'une valeur moyenne du débit cardiaque sur un temps long par rapport à la durée du cycle cardiaque.

Plusieurs techniques permettent actuellement la mesure du débit sanguin aortique chez les glires de laboratoire par le positionnement direct d'un capteur autour de l'artère aorte, également appelé bague aortique. Ce capteur est relié soit à un dispositif d'acquisition par un système filaire, auquel cas les mesures physiologiques de débit aortique sont réalisées sous anesthésie générale de l'animal, soit par un dispositif télémétrique qui permet une acquisition du débit aortique chez l'animal vigile, après une période de récupération post-opératoire. Les capteurs les plus utilisés sur les modèles glires jusque dans les années 2000 étaient les capteurs électromagnétiques [3-6] qui ont été progressivement remplacés par des capteurs dont le fonctionnement est basé sur la mesure de temps de transit ultrasonore (« transit time ultrasound technology » selon la terminologie anglo-saxonne) [7-10].

L'exploration non invasive de la fonction cardiaque chez le petit animal n'est réalisée aujourd'hui que par des méthodes (PET, IRM, CT scan, Echographie, Doppler pulsé) [11-14] extrêmement lourdes à mettre en oeuvre et contraignantes pour l'animal. En outre, certaines de ces méthodes (PET, CT scan et certains protocoles IRM) nécessitent un cathétérisme.

Une solution d'impédancemétrie basée sur des électrodes sous-cutanées (qui est donc invasive) [15] n'a pas donné lieu à développement commercial.

Il existe des solutions de télémétrie basées sur des systèmes implantables (et donc invasifs) comme le produit commercialisé par la société DSI™ [16].

Le document US 2008/255468 A1 décrit un vêtement équipé de capteurs de pléthysmographie par inductancemétrie pour un animal. De plus, le document US 5178151 A décrit des ceintures pour des mesures de pléthysmographie par inductancemétrie, à partir desquelles une pression aortique est déterminée.

**BREVE DESCRIPTION DE L'INVENTION**

Un but de l'invention est la mesure en continu du débit de l'aorte au niveau sous-diaphragmatique chez le petit mammifère de laboratoire, tel que le petit rongeur et le lagomorphe, de façon non invasive, non contraignante et en accord avec les contraintes réglementaires.

Conformément à l'invention, il est proposé un dispositif de mesure non-invasive du débit aortique au niveau sous-diaphragmatique chez un petit mammifère de laboratoire, le dispositif comprenant :

- un dispositif de pléthysmographie du thorax par inductancemétrie comportant deux spires extensibles électriquement conductrices solidaires d'un vêtement élastique ajustable au tronc dudit mammifère,
- un dispositif d'acquisition du signal de variation de section de chaque spire,
- un processeur configuré pour calculer le débit aortique sous-diaphragmatique instantané dudit mammifère, par extraction de la composante cardiaque des variations de volume du thorax à partir desdits signaux de variation de section de chaque spire et à partir d'un modèle fonctionnel du système cardio-respiratoire selon lequel les échanges de sang entre le thorax et le reste du corps dudit mammifère consistent en une sortie de sang par l'aorte abdominale

au niveau sous-diaphragmatique et en une entrée de sang par la veine cave inférieure.

Les performances métrologiques de cette mesure par pléthysmographie thoracique par inductance (PTI) sont dictées par les niveaux de volumes à mesurer, de quelques dizaines de microlitres pour les volumes sanguins chassés dans l'aorte à chaque battement cardiaque chez le petit animal, jusqu'à quelques millilitres chez les lagomorphes, et par les caractéristiques dynamiques de la fonction physiologique d'intérêt (fréquence cardiaque de 2 à 10 Hz, selon l'espèce). Ces contraintes métrologiques sont plus drastiques que celles dictées par la pléthysmographie respiratoire par inductance (PRI), pour laquelle les volumes sont de quelques centaines de millilitres et les fréquences physiologiques plus faibles (<1 Hz).

Selon d'autres caractéristiques avantageuses, considérées seules ou en combinaison :

- le diamètre de chaque spire à l'état libre est compris entre 2 et 15 cm ;
- l'espace entre lesdites spires est compris entre 0,5 et 5 cm ;
- les spires sont conformées en zigzag et, à l'état libre, la période spatiale des zigzags est comprise entre 0,5 et 1,5 cm ;
- l'amplitude desdits zigzags est comprise entre 0,5 et 3 cm.

Par « état libre » on entend l'état du dispositif de pléthysmographie lorsque le vêtement n'est pas porté par l'animal.

Par ailleurs, le dispositif peut en outre comprendre l'un au moins des éléments suivants :

- une spire solidaire dudit vêtement destinée à entourer l'abdomen du mammifère de sorte à effectuer une mesure de pléthysmographie respiratoire par inductancemétrie ;
- un capteur électrocardiographique solidaire dudit vêtement ;
- un accéléromètre solidaire dudit vêtement ;
- un microphone solidaire dudit vêtement, ledit microphone étant destiné à enregistrer les sons cardiaques du mammifère.

Un autre objet de l'invention concerne un procédé de mesure non-invasive du débit aortique au niveau sous-diaphragmatique chez un petit mammifère de laboratoire, le procédé comprenant :

- l'acquisition d'un signal de pléthysmographie thoracique par inductancemétrie au moyen de deux spires extensibles électriquement conductrices solidaires d'un vêtement élastique ajustable au tronc dudit mammifère,
- l'extraction, au moyen d'un processeur, à partir dudit signal pléthysmographique, de la composante cardiaque des variations de volume du thorax et la déduction du débit aortique sous-diaphragmatique sur la base d'un modèle fonctionnel du système cardio-respiratoire selon lequel les échanges de sang entre le thorax et le reste du corps dudit mammifère consistent en une sortie de sang par l'aorte abdominale au niveau sous-diaphragmatique et en une entrée de sang par la veine cave inférieure.

De manière particulièrement avantageuse, le débit aortique sous-diaphragmatique $D_{\text{Ao, sd}}$ peut être déterminé à partir de la formule :

$$D_{\text{Ao, sd}}(t) = - dV_{\text{bt}}(t) / dt + Q_c$$

où t est le temps, $V_{bt}(t)$ est la composante cardiaque des variations de volume du thorax et $Q_c$ est le débit cardiaque moyen dudit mammifère.

De manière avantageuse, on détermine le débit cardiaque moyen $Q_c$ en calculant la moyenne du signal $dV_{\text{bt}}(t)/dt$ sur le dernier tiers du cycle cardiaque dudit mammifère et en assignant ladite valeur moyenne au débit cardiaque moyen.

Selon des modes de réalisation mis en oeuvre séparément ou en combinaison :

- on acquiert en outre une mesure de pléthysmographie respiratoire par inductancemétrie dudit mammifère ;
- on acquiert en outre un électrocardiogramme dudit mammifère et l'on tient compte du signal électrocardiographique dans l'extraction de la composante cardiaque des variations de volume du thorax ;
- on acquiert en outre un signal accélérométrique représentatif d'une activité dudit mammifère et l'on tient compte dudit signal accélérométrique dans l'extraction de la composante cardiaque des variations de volume du thorax ;
- on enregistre en outre les sons cardiaques dudit mammifère au moyen d'un microphone et l'on tient compte dudit signal sonore dans l'extraction de la composante cardiaque des variations de volume du thorax.

Selon une forme d'exécution avantageuse, on met en oeuvre ledit procédé sur un mammifère vigile non contraint.

## BREVE DESCRIPTION DES DESSINS

D'autres caractéristiques et avantages de l'invention ressortiront de la description détaillée qui va suivre, en référence aux dessins annexés sur lesquels :

- la figure 1 est une représentation graphique du modèle physiologique fonctionnel mis en oeuvre dans l'invention,
- la figure 2 est une représentation graphique des signaux utilisés pour la détermination du débit aortique au niveau sous-diaphragmatique,
- la figure 3 est un schéma de principe d'un dispositif conforme à l'invention.

## DESCRIPTION DETAILLEE DE L'INVENTION

L'invention est basée sur la mise en oeuvre de la pléthysmographie du thorax et l'extraction, à partir de cette mesure, d'une variable fonctionnelle cardio-circulatoire : le débit de l'aorte sous-diaphragmatique.

Concernant la pléthysmographie, plusieurs technologies peuvent être mise en oeuvre. La technologie décrite ici est une technologie par inductancemétrie, qui est classiquement utilisée en pléthysmographie respiratoire par inductance (PRI) et appliquée ici aux variations de volume du thorax d'origine cardiaque et respiratoire (PTI).

Les sujets visés par une telle investigation fonctionnelle cardio-circulatoire sont des petits mammifères de laboratoire, tels que les rongeurs (souris, rats, cobayes, hamster, gerbille, furets) et les lagomorphes (lapins). D'une manière générale, ces animaux présentent une masse inférieure à 6000 g.

Pour la mise en oeuvre des mesures, l'animal peut être anesthésié ou vigile. Dans ce dernier cas, des mesures complémentaires sont nécessaires pour prendre en compte les artefacts liés à l'activité de l'animal.

Dans tous les cas, les mesures sont réalisées de manière non invasive, c'est-à-dire qu'elles ne nécessitent aucun rasage de l'animal ni intervention de nature chirurgicale.

Les activités cardiaque et respiratoire modifient les volumes du thorax et de l'abdomen du sujet.

En plaçant deux spires inductancemétriques à deux niveaux du thorax de l'animal, on peut estimer les variations de volume du thorax au cours du temps par une combinaison linéaire des variations de section estimées à ces deux niveaux par chacune des deux spires [17].

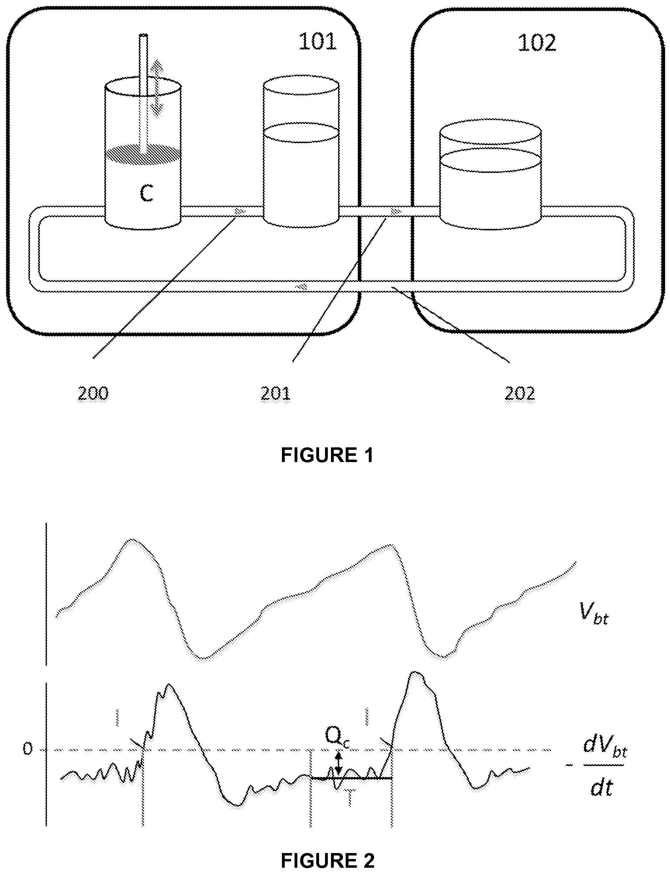

La figure 1 est une représentation graphique du modèle physiologique fonctionnel sur lequel est basé le calcul du débit aortique sous-diaphragmatique.

La pompe cardiaque C chasse le volume d'éjection systolique dans un ensemble de compartiments communicants : le compartiment thoracique 101 par l'aorte thoracique 200, puis le reste de l'organisme 102, principalement par l'aorte abdominale 201. Le retour du sang au thorax se fait par la veine cave inférieure 202.

Selon ce modèle, on considère que les variations de volume du thorax d'origine cardiaque sont liées à la chasse de sang hors du thorax par l'aorte abdominale pendant la systole (contraction du ventricule gauche) puis au retour veineux, qui est constant.

D'après le modèle physiologique fonctionnel de la figure 1, la composante cardiaque de la variation de volume du thorax, notée $V_{bt}$, est le résultat du déséquilibre dynamique entre le débit sanguin sortant du thorax et le débit sanguin y entrant.

Le modèle physiologique fonctionnel proposé réduit les échanges de sang entre le thorax et le reste du corps à ceux passant par l'aorte abdominale, au niveau sous-diaphragmatique (sortie) et la veine cave inférieure (entrée).

Si on définit les variables instantanées suivantes :

$D_{Ao, sd}(t)$ = débit aortique au niveau sous-diaphragmatique,
$D_{VCinf}(t)$ = débit de la veine cave inférieure au même niveau,

on peut écrire l'équation (1) reliant la dérivée selon le temps de la composante cardiaque du volume du thorax et les débits des deux vaisseaux considérés :

$$dV_{bt}(t) / dt = D_{VCinf}(t) - D_{Ao, sd}(t) \qquad (1)$$

Le débit de la veine cave est constant, égal au débit cardiaque moyen ($Q_c$), donc :

$$D_{Ao, sd}(t) = - dV_{bt}(t) / dt + Q_c \qquad (2)$$

L'estimation de $Q_c$ est rendue possible par le fait que le débit aortique au niveau sous-diaphragmatique s'annule pendant la dernière partie du cycle cardiaque.

La figure 2 est une représentation graphique des signaux utilisés dans le calcul, le signal $V_{bt}(t)$ et sa dérivée $dV_{bt}(t) / dt.$ Le signal $V_{bt}(t)$ augmente périodiquement en diastole et décroît brutalement à chaque systole.

Pour déterminer le débit aortique au niveau sous-diaphragmatique, on met en oeuvre, au moyen d'un processeur, un algorithme dont les différentes étapes sont les suivantes :

- détection du début de la phase d'éjection comme étant l'intersection I de [- $dV_{bt}(t) / dt$] avec l'axe des abscisses avant le pic positif d'amplitude maximale ;
- calcul de la moyenne du signal [$dV_{bt}(t) / dt$] sur le dernier tiers T du cycle ; et
- assignation de cette valeur à $Q_c$ ce qui permet le calcul de $D_{Ao, sd}(t)$ selon l'équation (2).

Le dispositif de mesure est un vêtement assimilable à un gilet qui est ajusté sur le corps de l'animal.

On fait en sorte que les deux spires, conformées en zigzag pour accompagner les déformations des sections qu'elles entourent, soient positionnées autour de deux régions du thorax de l'animal.

Le vêtement est avantageusement réalisé en un matériau extensible afin d'épouser étroitement le corps de l'animal. Le vêtement présente typiquement une forme tubulaire, avec éventuellement des trous pour le passage des pattes de l'animal. Les spires sont fixées sur le vêtement par exemple par couture.

Pour des animaux de cette taille, le diamètre des spires à l'état libre est typiquement compris entre 2 et 15 cm. Par ailleurs, l'espace entre les deux spires est typiquement compris entre 0,5 et 5 cm. En ce qui concerne l'amplitude et la période spatiale des zigzags des spires, elles sont respectivement de l'ordre de 0,5 à 5 cm et de 0,5 à 3 cm. Par rapport à la PRI, classiquement utilisée chez l'homme ou le gros animal, la valeur nominale de l'inductance des spires du présent dispositif destiné au petit mammifère de laboratoire est, compte tenu de sa taille, inférieure d'un facteur 10 environ.

Il existe différents modèles de vêtements et de configuration des spires selon la gamme de poids de l'animal et/ou l'espèce concernée.

Chaque spire fait partie d'un oscillateur dont on mesure la fréquence (qui est liée à la valeur d'inductance de la spire correspondante) au cours du temps.

A cet effet, on met en oeuvre un procédé de comptage.

Le contenu spectral de la composante cardiaque d'intérêt nécessite une fréquence d'échantillonnage de l'ordre de 100 Hz. Pour avoir une telle fréquence d'échantillonnage, il convient d'utiliser un outil de comptage rapide (de l'ordre de 200 MHz). De plus, cet outil doit être adaptatif pour faire face aux importants changements de section thoracique induits par les modifications posturales de l'animal.

Le dispositif de mesure comprend par ailleurs un processeur configuré pour mettre en oeuvre l'algorithme permettant d'extraire du signal $V_{bt}(t)$ le débit aortique au niveau sous-diaphragmatique selon l'équation 2.

Afin d'isoler la composante cardiaque du signal de variation de volume du thorax, la composante ventilatoire du signal doit être filtrée. Les techniques de filtrage conventionnelles avec une fréquence de coupure basse égale ou supérieure à la fréquence cardiaque moyenne ont été écartées au profit de méthodes numériques : approches en échelle (ondelettes, décomposition modale empirique) et/ou filtrages adaptatifs.

Par ailleurs, le dispositif de mesure doit être étalonné.

A cet effet, on réalise une mesure directe du débit aortique sous-diaphragmatique sur un animal de l'espèce à laquelle est destiné le dispositif. Une telle mesure directe peut par exemple être réalisée au moyen d'une bague aortique, qui est un dispositif implanté, au niveau sous-diaphragmatique, autour de l'aorte de l'animal et qui permet une mesure de débit par temps de transit des ultrasons. Des mesures de référence par gamme d'espèce et de poids sont réalisées sur des animaux anesthésiés équipés du dispositif. L'étalonnage du dispositif est obtenu par ajustement aux moindres carrés des paramètres de la combinaison linéaire d'estimation du débit aortique sous-diaphragmatique à partir des deux mesures de section.

Il est à noter qu'en raison de son caractère invasif un tel procédé d'étalonnage n'est pas adapté à l'homme. En revanche, chez le petit mammifère de laboratoire, une mesure directe du débit aortique sous-diaphragmatique peut être mise en oeuvre sur un animal d'une espèce donnée et utilisée pour étalonner le dispositif destiné à être utilisé sur les animaux de la même espèce.

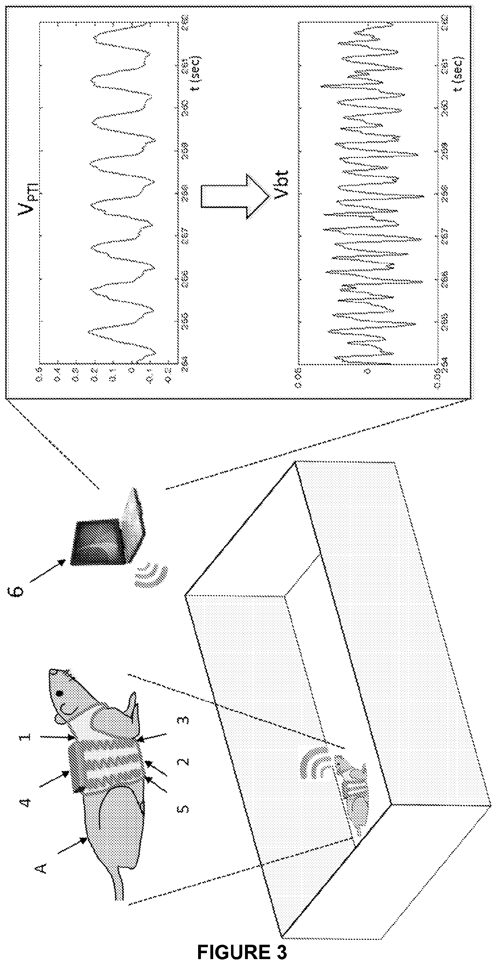

La figure 3 illustre un mode de réalisation permettant la mesure du débit aortique au niveau sous-diaphragmatique selon le procédé décrit ci-dessus.

Le gilet 1 est un dispositif non invasif permettant de préserver l'intégrité de l'animal A grâce à une mesure pléthysmographique par variation de l'inductance de deux spires 2, 3 localisées au niveau du thorax. Le gilet porte également un circuit électronique 4 destiné au conditionnement des signaux. Une spire supplémentaire 5 peut éventuellement être ajoutée au niveau abdominal pour permettre une mesure pléthysmographique d'ordre ventilatoire (PRI).

Le positionnement et la configuration des spires (amplitude et période spatiale des zigzags) sont déterminés par le modèle physiologique susmentionné et standardisé selon des critères morphologiques (gammes de poids, espèces concernées).

Le dispositif comprend en outre un processeur, faisant par exemple partie d'un ordinateur 6. Ledit processeur est configuré pour calculer, à partir des signaux de variation de section des spires, la variation de volume thoracique (signal $V_{PTI}$) et en extraire la composante cardiaque (signal $V_{bt}$) à partir de laquelle est déterminé le débit aortique sous-diaphragmatique.

De manière particulièrement avantageuse, les données de pléthysmographie sont transmises en continu au processeur par télémétrie. Cela permet en particulier de minimiser la contrainte imposée à l'animal, voire de mettre en oeuvre l'invention sur un animal vigile non contraint. Cependant, l'invention n'est pas limitée à cette forme d'exécution et peut comporter, le cas échéant, une liaison filaire entre le dispositif pléthysmographique et le processeur.

Eventuellement, le gilet peut porter un ou plusieurs autres capteurs que le dispositif pléthysmographique cardio-circulatoire.

Ainsi, notamment lorsque l'animal est vigile et non contraint, le gilet peut porter un accéléromètre permettant de prendre en compte les artefacts liés à l'activité de l'animal dans l'algorithme d'extraction. Les données accélérométriques sont utilisées dans le filtrage des artefacts de mouvement (référence bruit).

Par ailleurs, le gilet peut en outre porter un capteur électrocardiographique.

Enfin, le gilet peut également porter un microphone adapté pour enregistrer les sons cardiaques de l'animal. Les données électrocardiographiques, sonocardiographiques et PRI permettent, en outre, d'intégrer au dispositif une exploration cardio-respiratoire.

## REFERENCES

[1] McDonald D.A., "The velocity of blood flow in the rabbit aorta studied with high-speed cinematography". J. Physiol. 1952, 118:328-339.

[2] Spencer M.P. and Denison A.B., "The Aortic Flow Pulse as Related to Differential Pressure". Circ. Res. 1956, 4:476-484.

[3] US 2,808,723

[4] US 3,592,187

[5] US 4,346,604

[6] Berthonneche C., Sulpice T., Boucher F., Gouraud L., de Leiris J., O'Connor S.E., Herbert J.M. and Janiak P., "New insights into the pathological role of TNF-$\alpha$ in early cardiac dysfunction and subséquent heart failure following myocardial infarction in rats" Am. J. Physiol. 2004, 287:H340-H350.

[7] US 4,337,667

[8] US 6,098,466

[9] US 7,469,598

[10] Hoffman A., Grossman E., Ohman K.P., Marks E. and Harry R. Keiser H.R., "Endothelin Induces An Initial Increase in Cardiac Output Associated with Selective Vasodilation in Rats" Life Sci. 1989, 45(3):249-255.

[11] Xiong G., Paul C., Todica A., Hacker M., Bartenstein P. and Böning G., "Noninvasive image derived heart input function for CMRglc measurements in small animal slow infusion FDG PET studies". Phys. Med. Biol. 2012, 7;57(23):8041-59. doi: 10.1088/0031-9155/57/23/8041. Epub 2012 Nov 16. PubMed PMID: 23160517.

[12] Bible E., Dell'Acqua F., Solanky B., Balducci A., Crapo P.M., Badylak S.F., Ahrens E.T. and Modo M., "Non-invasive imaging of transplanted human neural stem cells and ECM scaffold remodeling in the stroke-damaged rat brain by (19)F- and diffusion-MRI". Biomaterials. 2012, 33(10):2858-71.

[13] Dinkel J., Bartling S.H., Kuntz J., Grasruck M., Kopp-Schneider A., Iwasaki M., Dimmeler S., Gupta R., Semmler W., Kauczor H.U. and Kiessling F., "Intrinsic gating for small-animal computed tomography: a robust ECG-less paradigm for deriving cardiac phase information and functional imaging". Circ. Cardiovasc. Imaging. 2008, 1(3):235-43.

[14] Nakamura T, Matsumuro A, Kuribayashi T, Matsubara K, Shima M, Shimoo K, Katsume H, Nakagawa M., "Echocardiographic détermination of stroke volume during rapid atrial pacing and volume loading in normal rats". Cardiovasc. Res. 1992, 26(8):765-9.

[15] Gotshall R.W., Breay-pilcher J.C. and Boelcskevy B.D., "Cardiac output in adult and neonatal rats utilizing impedance cardiography". Am. J. Physiol. 1987, 253(22): H1298-H1304.

[16] http://www.datasci.com/products/implantable-telemetry/

[17] Konno K. and Mead J. "Measurement of separate volume changes of rib cage and abdomen during breathing". J. Applied Physiol. 1967, 22(3):407-22.

**EP 3 340 859 B1**

**Revendications**

1. Dispositif de mesure non-invasive du débit aortique au niveau sous-diaphragmatique chez un petit mammifère de laboratoire, en particulier vigile et non contraint, le dispositif comprenant :

   a. un dispositif de pléthysmographie du thorax par inductancemétrie comportant deux spires extensibles (2, 3) électriquement conductrices solidaires d'un vêtement élastique (1) ajustable au tronc dudit mammifère (A),
   b. un dispositif (4) d'acquisition du signal de variation de section de chaque spire,
   c. un processeur (6) configuré pour calculer le débit aortique sous-diaphragmatique instantané dudit mammifère, par extraction de la composante cardiaque des variations de volume du thorax à partir desdits signaux de variation de section de chaque spire et à partir d'un modèle fonctionnel du système cardio-respiratoire selon lequel les échanges de sang entre le thorax et le reste du corps dudit mammifère consistent en une sortie de sang par l'aorte abdominale au niveau sous-diaphragmatique et en une entrée de sang par la veine cave inférieure.

2. Dispositif selon la revendication 1, dans lequel le diamètre de chaque spire (2, 3) à l'état libre est compris entre 2 et 15 cm et/ou l'espace entre lesdites spires est compris entre 0,5 et 5 cm.

3. Dispositif selon l'une des revendications 1 et 2, dans lequel les spires (2, 3) sont conformées en zigzag et, à l'état libre, la période spatiale des zigzags est comprise entre 0,5 et 1 ,5 cm et/ou l'amplitude desdits zigzags est comprise entre 0,5 et 3 cm.

4. Dispositif selon l'une des revendications 1 à 3, comprenant en outre une spire (5) solidaire dudit vêtement (1) destinée à entourer l'abdomen du mammifère (A) de sorte à effectuer une mesure de pléthysmographie respiratoire par inductancemétrie.

5. Dispositif selon l'une des revendications 1 à 4, comprenant en outre un capteur électrocardiographique solidaire dudit vêtement.

6. Dispositif selon l'une des revendications 1 à 5, comprenant en outre un accéléromètre solidaire dudit vêtement.

7. Dispositif selon l'une des revendications 1 à 6, comprenant en outre un microphone solidaire dudit vêtement, ledit microphone étant destiné à enregistrer les sons cardiaques du mammifère.

8. Procédé de mesure non-invasive du débit aortique au niveau sous- diaphragmatique chez un petit mammifère de laboratoire, le procédé comprenant :

   - l'acquisition d'un signal de pléthysmographie thoracique par inductancemétrie au moyen de deux spires extensibles (2, 3) électriquement conductrices solidaires d'un vêtement élastique (1) ajustable au tronc dudit mammifère (A),
   - l'extraction, au moyen d'un processeur (6), à partir dudit signal pléthysmographique, de la composante cardiaque des variations de volume du thorax et la déduction du débit aortique sous-diaphragmatique sur la base d'un modèle fonctionnel du système cardio-respiratoire selon lequel les échanges de sang entre le thorax et le reste du corps dudit mammifère consistent en une sortie de sang par l'aorte abdominale au niveau sous-diaphragmatique et en une entrée de sang par la veine cave inférieure.

9. Procédé selon la revendication 8, **caractérisé en ce que** le débit aortique sous-diaphragmatique ($D_{Ao,sd}$) est déterminé à partir de la formule :

$$D_{Ao,\,sd}(t) = - dV_{bt}(t) \, / \, dt + Q_c$$

où t est le temps, $V_{bt}(t)$ est la composante cardiaque des variations de volume du thorax et $Q_c$ est le débit cardiaque moyen dudit mammifère.

10. Procédé selon la revendication 9, dans lequel on détermine le débit cardiaque moyen ($Q_c$) en calculant la moyenne du signal $dV_{bt}(t)/dt$ sur le dernier tiers (T) du cycle cardiaque dudit mammifère et en assignant ladite valeur moyenne au débit cardiaque moyen.

7

**11.** Procédé selon l'une des revendications 8 à 10, dans lequel on acquiert en outre une mesure de pléthysmographie respiratoire par inductancemétrie dudit mammifère.

**12.** Procédé selon l'une des revendications 8 à 11, dans lequel on acquiert en outre un électrocardiogramme dudit mammifère et l'on tient compte du signal électrocardiographique dans l'extraction de la composante cardiaque des variations de volume du thorax.

**13.** Procédé selon l'une des revendications 8 à 12, dans lequel on acquiert en outre un signal accélérométrique représentatif d'une activité dudit mammifère et l'on tient compte dudit signal accélérométrique dans l'extraction de la composante cardiaque des variations de volume du thorax.

**14.** Procédé selon l'une des revendications 8 à 13, dans lequel on enregistre en outre les sons cardiaques dudit mammifère au moyen d'un microphone et l'on tient compte dudit signal sonore dans l'extraction de la composante cardiaque des variations de volume du thorax.

**15.** Procédé selon l'une des revendications 8 à 14, **caractérisé en ce qu'**il est mis en oeuvre sur un mammifère vigile non contraint.

**Patentansprüche**

**1.** Nichtinvasive Messvorrichtung der Durchflussrate durch die Aorta auf subdiaphragmatischem Niveau bei einem kleinen, insbesondere wachsamen und nicht eingeschränkten, Laborsäuger, wobei die Vorrichtung umfasst:

a. eine Vorrichtung der Plethysmographie des Thorax durch Induktanzmessung, umfassend zwei dehnbare elektrisch leitfähige Spiralen (2, 3), einstückig mit einem elastischen Anzug (1), der an den Rumpf des Säugetiers (A) anpassbar ist,
b. eine Vorrichtung (4) zum Erfassen des Abschnittsvariationssignals jeder Spirale,
c. einen Prozessor (6), konfiguriert zum Berechnen der momentanen subdiaphragmatischen Durchflussrate der Aorta des Säugetiers durch Extraktion des kardialen Bestandteils der Volumenvariationen des Thorax ausgehend von den Abschnittsvariationssignalen jeder Spirale und ausgehend von einem funktionalen Modell des Herz-Atmungs-Systems, wobei der Blutaustausch zwischen dem Thorax und dem Rest des Körpers des Säugetiers aus einem Austritt des Bluts durch die abdominale Aorta auf subdiaphragmatischen Niveau und einem Eintritt des Bluts durch die untere Hohlvene besteht.

**2.** Vorrichtung nach Anspruch 1, wobei der Durchmesser von jeder Spirale (2, 3) im freien Zustand im Bereich zwischen 2 und 15 cm liegt und/oder der Raum zwischen den Spiralen im Bereich zwischen 0,5 und 5 cm liegt.

**3.** Vorrichtung nach einem der Ansprüche 1 und 2, wobei die Spiralen (2, 3) im Zickzack ausgebildet sind und die räumliche Periode der Zickzacks im freien Zustand im Bereich zwischen 0,5 und 1,5 cm liegt und/oder die Amplitude der Zickzacks im Bereich zwischen 0,5 und 3 cm liegt.

**4.** Vorrichtung nach einem der Ansprüche 1 bis 3, umfassend des Weiteren eine mit dem Anzug (1) einstückige Spirale (5), dazu bestimmt, das Abdomen des Säugetiers (A) auf eine Weise zu umgeben, um eine Atmungsplethysmographiemessung durch Induktanzmessung durchzuführen.

**5.** Vorrichtung nach einem der Ansprüche 1 bis 4, umfassend des Weiteren einen mit dem Anzug einstückigen elektrokardiographischen Sensor.

**6.** Vorrichtung nach einem der Ansprüche 1 bis 5, umfassend des Weiteren einen mit dem Anzug einstückigen Beschleunigungsmesser.

**7.** Vorrichtung nach einem der Ansprüche 1 bis 6, umfassend des Weiteren ein mit dem Anzug einstückiges Mikrofon, wobei das Mikrofon dazu bestimmt ist, die kardialen Geräusche des Säugetiers aufzuzeichnen.

**8.** Nichtinvasives Messverfahren der Durchflussrate durch die Aorta auf subdiaphragmatischem Niveau bei einem kleinen Laborsäuger, wobei das Verfahren umfasst

- das Erfassen eines Thoraxplethysmographiesignals durch Induktanzmessung mittels zweier dehnbaren elektrisch leitfähigen Spiralen (2, 3), einstückig mit einem elastischen Anzug (1), der an den Rumpf des Säugetiers (A) anpassbar ist,

- die Extraktion, mittels eines Prozessors (6), ausgehend von dem Plethysmographiesignal, des kardialen Bestandteils der Volumenvariationen des Thorax und die Deduktion der subdiaphragmatischen Durchflussrate durch die Aorta auf der Basis eines funktionalen Modells des Herz-Atmungs-Systems, wobei der Blutaustausch zwischen dem Thorax und dem Rest des Körpers des Säugetiers aus einem Austritt des Bluts durch die abdominale Aorta auf subdiaphragmatischen Niveau und einem Eintritt des Bluts durch die untere Hohlvene besteht.

9. Verfahren nach Anspruch 8, **dadurch gekennzeichnet, dass** die subdiaphragmatische Durchflussrate durch die Aorta ($D_{Ao,sd}$) bestimmt wird ausgehend von der Formel:

$$D_{Ao,sd}(t) = - dV_{bt}(t) \, / \, dt + Q_c$$

wobei t die Zeit ist, $V_{bt}(t)$ der kardiale Bestandteil der Volumenvariationen des Thorax ist und $Q_c$ die mittlere kardiale Durchflussrate des Säugetiers ist.

10. Verfahren nach Anspruch 9, wobei man die mittlere kardiale Durchflussrate ($Q_c$) bestimmt, indem der Mittelwert des Signals $dV_{bt}(t)/dt$ auf dem letzten Drittel (T) des kardialen Zyklus des Säugetiers berechnet wird und indem der mittlere Wert der mittleren kardialen Durchflussrate zugeordnet wird.

11. Verfahren nach einem der Ansprüche 8 bis 10, wobei man des Weiteren eine Atmungsplethysmographiemessung durch Induktanzmessung des Säugetiers erfasst.

12. Verfahren nach einem der Ansprüche 8 bis 11, wobei man des Weiteren ein Elektrokardiogramm des Säugetiers erfasst und man das elektrokardiographische Signal bei der Extraktion des kardialen Bestandteils der Volumenvariationen des Thorax berücksichtigt.

13. Verfahren nach einem der Ansprüche 8 bis 12, wobei man des Weiteren ein beschleunigendes Signal, das repräsentativ ist für die Aktivität des Säugetiers, erfasst und man das beschleunigendes Signal bei der Extraktion des kardialen Bestandteils der Volumenvariationen des Thorax berücksichtigt.

14. Verfahren nach einem der Ansprüche 8 bis 13, wobei man des Weiteren die kardialen Geräusche des Säugetiers mittels eines Mikrofons aufzeichnet und man das akustische Signal bei der Extraktion des kardialen Bestandteils der Volumenvariationen des Thorax berücksichtigt.

15. Verfahren nach einem der Ansprüche 8 bis 14, **dadurch gekennzeichnet, dass** es an einem wachsamen, nicht eingeschränkten Säugetier durchgeführt wird.

**Claims**

1. Device for non-invasively measuring the aortic flow at the sub-diaphragmatic level of a small laboratory mammal, in particular awake and non-constrained, the device comprising:

   a. a plethysmographic inductance meter device for the thorax comprising two electrically conductive extendible spires (2, 3) secured to an elastic garment (1) adjustable at the trunk of said mammal (A),
   b. a device (4) for acquiring the section variation signal of each spire,
   c. a processor (6) configured to calculate the instantaneous sub-diaphragmatic aortic flow of said mammal, by extracting the cardiac component of the variations in volume of the thorax from said section variation signals of each spire and from a functional model of the cardio-respiratory system according to which the blood exchanges between the thorax and the remainder of the body of said mammal consist of a blood outlet through the abdominal aorta at the sub-diaphragmatic level and of a blood inlet through the inferior vena cava.

2. Device according to claim 1, wherein the diameter of each spire (2, 3) in the free state is between 2 and 15cm and/or the space between said spires is between 0.5 and 5cm.

3. Device according to one of claims 1 and 2, wherein the spires (2, 3) are zigzag-shaped and, in the free state, the spatial period of the zigzags is between 0.5 and 1.5cm and/or the amplitude of said zigzags is between 0.5 and 3cm.

4. Device according to one of claims 1 to 3, further comprising a spire (5) secured to said garment (1) intended to surround the abdomen of the mammal (A) so as to take a respiratory plethysmographic measurement by an inductance meter.

5. Device according to one of claims 1 to 4, further comprising an electrocardiographic sensor secured to said garment.

6. Device according to one of claims 1 to 5, further comprising an accelerometer secured to said garment.

7. Device according to one of claims 1 to 6, further comprising a microphone secured to said garment, said microphone being intended to record the cardiac sounds of the mammal.

8. Method for non-invasively measuring the aortic flow at the sub-diaphragmatic level in a small laboratory mammal, the method comprising

- the acquisition of a thoracic plethysmographic signal with an inductance meter by means of two electrically conductive extendible spires (2, 3) secured to an elastic garment (1) adjustable at the trunk of said mammal (A),
- the extraction, by means of a processor (6), from said plethysmographic signal, of the cardiac component of the variations in volume of the thorax and the deduction of the sub-diaphragmatic aortic flow based on a functional model of the cardio-respiratory system according to which the blood exchanges between the thorax and the remainder of the body of said mammal consist of a blood outlet through the abdominal aorta at the sub-diaphragmatic level and of a blood inlet through the inferior vena cava.

9. Method according to claim 8, **characterised in that** the sub-diaphragmatic aortic flow ($D_{Ao,sd}$) is determined from the formula:

$$D_{AO,\,sd}(t) = -\,dV_{bt}(t)\,/\,dt + Q_c$$

where t is time, $V_{bt}(t)$ is the cardiac component of the variations in volume of the thorax, and $Q_c$ is the average cardiac flow of said mammal.

10. Method according to claim 9, wherein the average cardiac flow ($Q_c$) is determined by calculating the average of the signal $dV_{bt}(t)/dt$ over the last third (T) of the cardiac cycle of said mammal and by assigning said average value to the average cardiac flow.

11. Method according to one of claims 8 to 10, wherein a respiratory plethysmographic measurement with an inductance meter is furthermore acquired from said mammal.

12. Method according to one of claims 8 to 11, wherein an electrocardiogram of said mammal is furthermore acquired, and the electrocardiographic signal is taken into account in extracting the cardiac component from the variations in volume of the thorax.

13. Method according to one of claims 8 to 12, wherein an accelerometric signal is furthermore acquired, representative of an activity of said mammal and said accelerometric signal is taken into account in extracting the cardiac component from the variations in volume of the thorax.

14. Method according to one of claims 8 to 13, wherein the cardiac sounds of said mammal are furthermore recorded by means of a microphone and said sound signal is taken into account in extracting the cardiac component from the variations in volume of the thorax.

15. Method according to one of claims 8 to 14, **characterised in that** it is implemented on an awake, non-constrained mammal.

**FIGURE 1**

**FIGURE 2**

**FIGURE 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2008255468 A1
- US 5178151 A
- US 2808723 A
- US 3592187 A
- US 4346604 A
- US 4337667 A
- US 6098466 A
- US 7469598 B

**Littérature non-brevet citée dans la description**

- **MCDONALD D.A.** The velocity of blood flow in the rabbit aorta studied with high-speed cinematography. *J. Physiol.,* 1952, vol. 118, 328-339
- **SPENCER M.P ; DENISON A.B.** The Aortic Flow Pulse as Related to Differential Pressure. *Circ. Res.,* 1956, vol. 4, 476-484
- **BERTHONNECHE C. ; SULPICE T. ; BOUCHER F. ; GOURAUD L. ; DE LEIRIS J. ; O'CONNOR S.E. ; HERBERT J.M. ; JANIAK P.** New insights into the pathological role of TNF-$\alpha$ in early cardiac dysfunction and subséquent heart failure following myocardial infarction in rats. *Am. J. Physiol.,* 2004, vol. 287, H340-H350
- **HOFFMAN A. ; GROSSMAN E. ; OHMAN K.P. ; MARKS E. ; HARRY R ; KEISER H.R.** Endothelin Induces An Initial Increase in Cardiac Output Associated with Selective Vasodilation in Rats. *Life Sci.,* 1989, vol. 45 (3), 249-255
- **XIONG G. ; PAUL C. ; TODICA A. ; HACKER M. ; BARTENSTEIN P ; BÖNING G.** Noninvasive image derived heart input function for CMRglc measurements in small animal slow infusion FDG PET studies. *Phys. Med. Biol.,* 2012, vol. 7;57 (23), 8041-59
- **BIBLE E. ; DELL'ACQUA F. ; SOLANKY B. ; BALDUCCI A. ; CRAPO P.M. ; BADYLAK S.F. ; AHRENS E.T. ; MODO M.** Non-invasive imaging of transplanted human neural stem cells and ECM scaffold remodeling in the stroke-damaged rat brain by (19)F- and diffusion-MRI. *Biomaterials,* 2012, vol. 33 (10), 2858-71
- **DINKEL J. ; BARTLING S.H. ; KUNTZ J. ; GRASRUCK M. ; KOPP-SCHNEIDER A. ; IWASAKI M. ; DIMMELER S. ; GUPTA R. ; SEMMLER W. ; KAUCZOR H.U.** Intrinsic gating for small-animal computed tomography: a robust ECG-less paradigm for deriving cardiac phase information and functional imaging. *Circ. Cardiovasc. Imaging.,* 2008, vol. 1 (3), 235-43
- **NAKAMURA T ; MATSUMURO A ; KURIBAYASHI T ; MATSUBARA K ; SHIMA M ; SHIMOO K ; KATSUME H ; NAKAGAWA M.** Echocardiographic détermination of stroke volume during rapid atrial pacing and volume loading in normal rats. *Cardiovasc. Res.,* 1992, vol. 26 (8), 765-9
- **GOTSHALL R.W. ; BREAY-PILCHER J.C. ; BOELCSKEVY B.D.** Cardiac output in adult and neonatal rats utilizing impedance cardiography. *Am. J. Physiol.,* 1987, vol. 253 (22), H1298-H1304
- **KONNO K. ; MEAD J.** Measurement of separate volume changes of rib cage and abdomen during breathing. *J. Applied Physiol.,* 1967, vol. 22 (3), 407-22